# EUROPEAN PATENT SPECIFICATION

(11) **EP 2 942 050 B1**
(45) Date of publication and mention of the grant of the patent: **07.09.2022**
(21) Application number: 15166580.9
(22) Date of filing: 06.05.2015
(51) Int. Cl.: A61K 9/00, A61K 47/14, A61K 31/592, A61K 31/593

(54) **A SPRAYABLE OILY COMPOSITION BASED ON LIPOSOLUBLE VITAMINS OF THE GROUP D AND USE THEREOF**
ZERSTÄUBARE ÖLIGE ZUSAMMENSETZUNG AUF BASIS VON FETTLÖSLICHEN VITAMINEN AUS DER D GRUPPE UND VERWENDUNG DAVON
COMPOSITION HUILEUSE PULVÉRISABLE À BASE DE VITAMINES LIPOSOLUBLES DU GROUPE D ET SON UTILISATION

(30) Priority: 06.05.2014 IT MI20140824
(43) Date of publication of application: 11.11.2015
(73) Proprietor: S.I.I.T. S.r.l.-Servizio Internazionale Imballaggi Termosaldanti, 20090 Trezzano sul Naviglio MI (IT)
(72) Inventor: Costa, Andrea, 20090 Trezzano sul Naviglio (MI) (IT)
(74) Representative: Benedetto, Marco

(56) References cited:
- WO-A1-2008/031188
- WO-A1-2012/117236
- CN-A- 1 732 958
- US-A1- 2005 281 749
- US-A1- 2008 019 957
- US-A1- 2010 221 350

## Description

The present invention relates to a process for preparing a sprayable oily liquid formulation.

Based on their solubility, vitamins can be divided and classified into liposoluble and hydrosoluble vitamins.

Among liposoluble vitamins the following can be mentioned by way of example: vitamin A (retinol and analogs thereof), vitamin D (ergocalciferol D2 and cholecalciferol D3), vitamin E (tocopherol), vitamin K (naphthoquinone and derivatives thereof), vitamin F (alpha-linolenic acid and derivatives thereof) and vitamin Q (ubiquinone).

There are many formulations based on one or more liposoluble and/or hydrosoluble vitamins on the market, which formulations can be in solid form for oral use, such as e.g. as tablets, capsules or granules, or in liquid form, such as e.g. as solutions in drops or suspensions in syrups. Until today there are no formulations on the market based on liposoluble vitamins, preferably vitamin D, in sprayable liquid form due to their liposoluble nature.

An effective administration of one or more vitamins depends on various factors such as the administration route, the pharmaceutical form used, the absorption at the oral mucosa and/or at the intestine, the vitamin bioavailability and the metabolic changes occurring.

For instance, vitamin D is present in two main forms, i.e. as vitamin D2 (ergocalciferol) and as vitamin D3 (cholecalciferol). Both vitamin D2 and vitamin D3 are two inactive provitamins and should be subjected to two enzymatic hydroxylation reactions so as to be turned into the final, biologically active form, i.e. calcitriol (1,25-dihydroxyvitamin D; 1,25(OH)D). Recent studies have shown that vitamin D3 is more available and easily digestible than vitamin D2 and that at the same dosage oily formulations are more effective than capsules since they increase further the serum levels of 25(OH)D.

A deficiency of vitamin D3 can affect human health in various ways. There are at present oily solutions of vitamin D in drops, but their use has some limiting drawbacks. For instance, in the case of children of pediatric and adolescent age the administration of an oily solution in drops of vitamin D3 is not always easy and/or possible. Moreover, oily solutions are hardly accepted since in drop form they have an unpleasant taste and a bad palatability as well as an oily feeling in the mouth.

WO 2008/031188 A1 discloses a composition of vitamin D in medium-chain triglycerides and use thereof in delivering a nutritional or therapeutic amount of vitamin D to a human being, particularly, an infant.

However, the attempts made in order to reduce the above mentioned limitations affecting oily solutions in drop form were directed to the development of solid or aqueous dispersions of vitamin D, but these attempts resulted in a reduction of the bioavailability of the vitamin itself which was less absorbable than in a solution.

Therefore, skilled technicians are still in need of new formulations and pharmaceutical forms for administration that enable an effective administration of one or more liposoluble vitamins, such as e.g. vitamin D. Skilled technicians are further in need of new formulations and preparation methods that are easily implemented, repeatable and low-cost.

After long and deep research and development activities, the Applicant has found an answer to the above-mentioned needs. The Applicant has developed a new formulation comprising one or more liposoluble vitamins and a new pharmaceutical form of administration for dispensing repeatable and precise doses so as to ensure a greater ease of use.

An object of the present description is a sprayable oily liquid formulation comprising one or more liposoluble vitamins having the characteristics as claimed in the appended independent claim.

An object of the present description is a sprayable oily liquid formulation comprising one or more liposoluble vitamins for use as a food supplement or a medical device or a pharmaceutical composition for use in treating subjects suffering from diseases and/or disorders associated to vitamin lacks or deficiencies, having the characteristics as claimed in the appended independent claim.

An object of the present invention is a process for preparing a sprayable oily liquid formulation having the characteristics as claimed in appended independent claim 1.

Other preferred embodiments are disclosed in the following detailed description.

It is known that oils are generally little or hardly compressible to be used in a spray administration system.

The Applicant has found that it is possible to prepare a stable oily liquid formulation which can be subjected to compression since it is compressible under pressure. The possibility of easily compressing the liquid formulation of the present description has led to the development of a new pharmaceutical form of administration of liposoluble vitamins, such as vitamin D, with a spray system.

Advantageously, the formulation of the present description, which can be administered orally by means of a spray system, is not characterized by the so-called oily feeling thanks to the fact that a drop of said oily liquid formulation can be highly divided into a homogeneous cloud of very thin particles.

Advantageously, by adopting a suitable spray system it is possible to easily administer - wherever the patient - with two 50 µl puffs a repeatable and precise dose of 400 IUs of vitamin D, preferably vitamin D3, which represents the recommended daily amount (RDA). This repeatable and precise dosage allows to administer one or more liposoluble vitamins of group D, such as vitamin D3, to all subjects without adverse effects or contraindications.

The oily liquid formulation of the present description, containing at least one liposoluble vitamin of group D, such as vitamin D3, can be subjected to compression, since it is compressible under pressure, thanks to the use of a compound (food ingredient) that is acceptable from a dietary, nutraceutical and pharmaceutical point of view, represented by triglycerides of medium chain acids, also referred to as CAS 73398-61-5 or 85409-09-2 or 438544-49-1; or number EINECS 277-452-2; or also referred to with the chemical name of glycerides, mixed decanoyl and octanoyl, or C8-C10 glycerides.

Said triglyceride of medium chain acids is chosen among triglycerides having the following characteristics:
- caprylic acid (C8) 50 to 80%; preferably at least 59%, and
- capric acid (C10) 20 to 50%; preferably at least 40%.

Advantageously, the sum of saturated fatty acids (C8+C10) is of 96% or above, preferably of 98% or above or above 99.5%.

Said triglyceride should also have a higher amount of caprylic acid (C8) than capric acid (C10) of a weight amount of 10 to 40%, preferably of 15 to 35%. Said triglycerides of medium chain acids also have:
- a viscosity of 20°C: 25 to 33 mPa.s,
- an acid number: 0.20 mg KOH/g or below,
- a saponification value: 310 to 360 mg KOH/g,
- a water content: 0.20% or below.

Advantageously, said triglyceride of medium chain acids is a triglyceride of capric acid (C10) and/or caprylic acid (C8) having the commercial name of Labrafac Lipophile WL 1349 Code 3139 (European Pharmacopeia no. 0868) of the company Gattefossé SAS.

In a preferred embodiment, the composition of said triglyceride comprises or alternatively consists of:
- caproic acid C6: 2% or below, e.g. below 0,1%;
- caprylic acid C8: 50% to 80%, e.g. 59.2%;
- capric acid C10: 20% to 50%, e.g. 40.5%;
- lauric acid C10: 3% or below, e.g. 0.2%;
- myristic acid C14: 1% or below, e.g. below 0.1%.

The formulation of the present description comprises a mixture comprising or alternatively consisting of a liposoluble vitamin and triglycerides of medium chain acids, as defined above.

Advantageously, the liposoluble vitamin is of group D, such as vitamin D3, and said triglyceride of medium chain acids is Labrafac Lipophile WL 1349.

The mixture further comprises a medium chain triglyceride oil (MCT Oil) in association with said triglyceride of medium chain acids. Moreover, the formulation can also comprise excipients, sweeteners and flavorings.

The medium chain triglyceride oil - MCT oil - can be of the type Miglyol 810 or 812 or Crodamol GTC/C and has a composition of 1-3% of fatty acids C6 and of 1-5% of fatty acids C12; these are obtained by hydrolysis of coconut oil followed by fractionation of the fatty acids. The medium chain triglyceride oil (MCT Oil) used here has a lower content of C8 and/or C10 than the medium chain triglyceride used in the present invention. The medium chain triglyceride oil (MCT Oil) has a lower purity than the triglycerides of medium chain acids used here, however the MCT oil is far cheaper than said triglycerides of medium chain acids. The lower purity is also related to the fact that the MCT oil has a much higher lipase content than the content in said triglycerides such as Labrafac Lipophile WL 1349.

Lipases are responsible for oil hydrolysis, in particular for the hydrolysis of the triglycerides present in the MCT oil. Oil hydrolysis by lipases leads to a worse palatability since there is an increase of the so-called oil feeling, furthermore the reduction of the amount of triglycerides in the oil, due to the hydrolysis by lipases, negatively affects the quality of atomization of the formulation of the present description. In order to obviate this drawback, the Applicant has found to use a 1:1 weight ratio of the amount of triglycerides of medium chain acids, such as Labrafac Lipophile WL 1349, to the amount of medium chain triglyceride oil (MCT oil), such as Miglyol 810 or 812 or Crodamol GTC/C, in combination with a heating step for the medium chain triglyceride oil before it gets in contact or is mixed with vitamin D, such as vitamin D3, and/or the triglyceride of medium chain acids, such as Labrafac Lipophile WL 1349.

In one embodiment, the liposoluble vitamin is vitamin D, vitamin D2 and/or vitamin D3, the triglyceride of medium chain acid is a triglyceride of capric and/or caprylic acid (C8-C10), preferably Labrafac Lipophile WL 1349 Code 3139 (European Pharmacopeia no. 0868) of the company Gattefossé SAS, and the medium chain triglyceride oil is preferably Miglyol 810 or 812 or Crodamol GTC/C.

The formulation of the present description, in the form of an oily solution, has a density in the range of 0.92 to 0.98 g/cm³ at 25°C; preferably of 0.94 to 0.96 g/cm³ to 25°C.

The medium chain triglyceride oil and the triglyceride of medium chain acids are present in the mixture contained in the formulation of the present description in a weight ratio of 1:20 to 1:10, preferably of 1:20 to 1:5; advantageously of about 1:1. The weight ratio of about 1:1 allows to prepare a stable, effective and very cheap formulation.

An object of the present description is a formulation comprising a mixture comprising or alternatively consisting of a liposoluble vitamin of group D, such as vitamin D3, a triglyceride of medium chain acids, such as Labrafac Lipophile WL 1349, a medium chain triglyceride oil (MCT oil), such as Miglyol 810 or 812 or Crodamol GTC/C, the latter two being present in said mixture in a weight ratio of about 1:1.

An object of the present invention is a process for preparing a sprayable oily formulation comprising a mixture comprising, or alternatively consisting of: (a) one or more liposoluble vitamins of group D, (b) a triglyceride of medium chain acids chosen among triglycerides of capric acid and/or caprylic acid, and (c) a medium chain triglyceride oil (MCT oil) having a lower content of C8 and/or C10 fatty acids than that of (b). Said process comprises a step in which the liposoluble vitamin of group D, such as vitamin D3, is dissolved in a medium chain triglyceride oil heated to a temperature of 40°C to 60°C, preferably of 45°C to 55°C, e.g. 50°C, under stirring until the disappearance of the bottom body so that to obtain an oily solution and vitamin. Then the triglyceride of medium chain acids, such as Labrafac Lipophile WL 1349, is added to said oily solution and vitamin. Finally, the whole is cooled to a room temperature of about 20°C under stirring. Said medium chain triglyceride oil and said triglyceride of medium chain acids are present in a weight ratio of about 1:1.

In another embodiment of the present description, the triglyceride of medium chain acids, such as Labrafac Lipophile WL 1349, is added directly to the liposoluble vitamin of group D and then a medium chain triglyceride oil heated to a temperature of 40°C to 60°C, preferably of 45°C to 55°C, e.g. 50°C, is added under stirring. Finally, the whole is cooled to a room temperature of about 20°C under stirring. Then, if desired, flavorings and sweeteners can be added before dividing the formulation thus obtained into bottles, preferably 10 ml bottles.

Below are listed some experimental data obtained by the Applicant on an exemplary formulation of the present description (Composition C: medium chain triglyceride oil, MCT Oil, such as Miglyol 810 (23.647 mg); triglycerides of medium chain acids, such as Labrafac Lipophile WL 1349 (23.647 mg) and crystalline vitamin D3 (0.0060 mg)). The spraying tests were made with an apparatus having the following characteristics:
Container: item no. H92/15
Capacity: 10 ml
Material: glass
Filling method: manual

Drop size distribution analysis:
Analysis made at room temperature (22.5±1 °C) on 5 pumps, carrying out 2 tests for each pump.
Pump: PUMP-50-VP6-THREAD-PFP18-1R-26,0
Actuator: ACT_ASM-161C-15,10-M-3,90-5,34-WHIT

Protocol: Charge the pump with at least 5 complete sprays. Measure the drop size distribution with a laser diffraction instrument (MASTERSIZER S of MALVERN SQ203; software: 2.19; sweep no.: 75; radiation wavelength: 2.40 nm; lens/actuator distance: 70 mm; laser beam/actuator distance:40 mm; pump position: 90 degrees.

Calculate the following average values:
D(v, 0.1): indicates the value of the diameter in which 10% of the particle lies;
D (v, 0.5): indicates the value of the diameter in which 50% of the particle lies;
D (v, 0.9): indicates the value of the diameter in which 90% of the particle lies;
% <10µm: indicates the percentage of particles having a diameter below 10 µm.

A first sample A of said composition C was prepared as follows: crystalline vitamin D3 (0.0060 mg) is dissolved in a medium chain triglyceride oil such as Miglyol 810 (23.647 mg) previously heated to a temperature of 50°C, under stirring until the disappearance of the bottom body so as to obtain an oily solution and vitamin. Then Labrafac Lipophile WL 1349 (23.647 mg) was added to said oily solution and vitamin. Finally, the final solution was cooled to a room temperature of about 20°C under stirring. The heating step deactivated the lipases present in the medium chain triglyceride oil and the lipases present in the surrounding outer medium. Thus, the amounts of triglycerides present in the oil did not change.

A second sample B of said composition C was prepared as sample A at a room temperature of 20°C (without heating steps).

The measurements on sample A (Table 1) and on sample B (Table 2) were made with the same apparatus and at a temperature of about 20°C.

Table 1 shows that, when Labrafac Lipophile WL 1349 and Miglyol 810 are used in a weight ratio of 1:1 and the percentage of triglycerides in the mixture is kept constant, since their hydrolysis by the lipases was prevented, the formulation can be atomized in a particularly advantageous manner. As a matter of fact, 10% of atomized particles have an average diameter of 37.13 µm, 50% of atomized particles have an average diameter of 126.7 µm, and 90% of atomized particles have an average diameter of 380.35 µm.

The better atomization of the product is related to a better palatability thereof thanks to the elimination of the so-called oil feeling, which as a result simplifies product administration also to children of pediatric and adolescent age. In the experimental tests attached hereto, the percentage of particles having a diameter below 10 µm was also measured. Said particles represent the breathable fraction of the sprayable formulation and it is therefore desirable that such a percentage remains very low. As can be inferred from Table 1, only 0.36% of particles have a diameter below 10 µm.

In Table 2 the same sample as in Table 1 was analyzed, but the formulation was obtained by mixing the components at room temperature, without any heating step.

Table 2 clearly shows that the analyzed sample has a worse atomization since the average diameter of the atomized particles is far larger than the sample of Table 1: 10% of atomized particles have an average diameter of 61.19 µm (vs. 37.13 µm of Table 1), 50% of atomized particles have an average diameter of 250.40 µm (vs. 126.7 µm of Table 1) and 90% of atomized particles have an average diameter of 456.03 µm (vs. 380.35 µm of Table 1). Moreover, the breathable particle fraction increases (0.45% instead of 0.36).

This difference in the atomization quality is due to the presence of lipases both in the medium chain triglyceride oil (MCT oil) and in the surrounding outer medium. The lipases hydrolyze the triglycerides with an increase of free fatty acids.

This information was further confirmed by experimental tests in which the acid number of 3 formulations having the same composition C was measured. The formulations were stored for 24 months at 25°C (see Table 3).

The acid number is a measure of the amount of sodium hydroxide expressed in milligrams required for neutralizing the free fatty acids present in one gram of substance. Therefore, an increase of the acid number is related to an increase of free fatty acids in a formulation.

Formulations obtained by heating the components at 45°C and at 50°C during preparation and formulations obtained without heating (at room temperature) were compared. The results are shown in Table 3.

Table 3 clearly shows that the acid number of the formulations obtained without heating is at least six times higher than the acid number of the formulations in which the lipases were deactivated. From this it can be inferred that, when the lipases are not deactivated, the percentage of triglycerides deriving from Labrafac and Miglyol decreases whereas the concentration of free fatty acids in the formulation increases. This change in the composition of the formulations leads to a worse atomization thereof, as shown in the spraying tests described above.

The use of such mixture is further advantageous in that it allows to reduce manufacturing costs with respect to a composition containing a triglyceride of medium chain acids only, chosen among the triglycerides of capric and caprylic acid of the type Labrafac WL 1349 Code 3139.

Therefore, the experimental data attached hereto show that the atomization quality of the particles depends on the percentage of triglycerides present in the composition as well as on the stability of said triglycerides to lipase-induced hydrolysis.

The Applicant tested its oily formulation in order to compare the plasma levels of 25 hydroxy-vitamin D (25(OH) D3) obtained by administering cholecalciferol vitamin D3. The comparison was made between two oral formulations: a formulation known on the market as drops for oral administration and an oily formulation of the present description as sublingual spray, having the following characteristics:
- density: 899.618 mg/ml
- volume: 1 puff 0.050 ml; mg/puff (50 µl puff = 47.30 mg)
- Composition C (total weight 47.30 mg): medium chain triglyceride oil - MCT Oil, such as Miglyol 810 (23.647 mg); triglycerides of medium chain acids, such as Labrafac Lipophile WL 1349 (23.647 mg), and crystalline vitamin D3 (0.0060 mg).

The experimental phase lasted for a total of 28 days during which the treatments took place according to a crossover design.
- 7 days of administration of the formulation in drops.
- 14 days of wash-out.
- 7 days of administration of the sublingual spray formulation of oily solution.

The doses are taken in the morning after breakfast:
- Orally administered drops: 1 dose of 400 IUs of cholecalciferol (10 mcg/die) in 5 drops diluted in a half glass of water.
- Sublingual spray of oily solution: 1 dose of 400 IUs of cholecalciferol (10 mcg/die) in two sublingual puffs.

The enrolled subjects were asked to keep their dietary habits, in particular concerning food with a significant content of vitamin D, and the exposition to sunrays constant during the periods covering their participation in the two evaluation phases.

For each of the administration periods, blood samples were taken for measuring the plasma levels of 25 (OH) D3 in the morning at a fasted state at the following times:
- T0: before the beginning of the first week of administration
- T1: after one day from the first administration
- T7: after seven days of administration

The experimental chemiluminescence method was used.

The study confirmed that the spray oily solution of the present description has supplied at least (if not more in some cases) the same plasma levels of 25(OH) D3 as the orally administered drops. Moreover, the bioavailability of vitamin D3 in the oily spray is comparable (if not better in some cases) to the one of vitamin D3 in drops.

From a compliance point of view, the subjects reported they liked the administration of the spray oily solution better than the orally administered drops since the first does not have a disagreeable taste and has an excellent palatability without any oily feeling both at the beginning of administration and after administration.

**Table 1**

| **Pump nr.** | ***D (v,0.1)*** | ***D (v*,*0.5)*** | ***D (v,0.9)*** | **% < *10µm*** |
|---|---|---|---|---|
| | *measure unit*: *µm* | | | |
| 1 | 34.16 | 130.51 | 388.05 | 0.27 |
| | 33.36 | 121.79 | 368.4 | 0.28 |
| 2 | 43.66 | 143.88 | 377.94 | 0.39 |
| | 40.78 | 132.88 | 373.69 | 0.47 |
| 3 | 40.79 | 140.96 | 380.39 | 0.46 |
| | 36.98 | 126.63 | 310.67 | 0.39 |
| 4 | 37.04 | 126.22 | 386.25 | 0.22 |
| | 35.66 | 129.57 | 391.58 | 0.24 |
| 5 | 34.29 | 110.58 | 385.24 | 0.40 |
| | 34.60 | 104.14 | 381.30 | 0.45 |
| Minimum | 33.38 | 104.14 | 368.40 | 0.22 |
| Maximum | 43.66 | 143.88 | 391.58 | 0.47 |
| **Average** | **37.13** | **126.7** | **380.35** | **0.36** |
| Std. Dev | 3.48 | 12.26 | 7.69 | 0.06 |

**Table 2**

| **Pump nr**. | ***D (v,0.1)*** | ***D (v,0.5)*** | ***D (v,0.9)*** | **%** < ***10µm*** |
|---|---|---|---|---|
| | *measure unit: µm* | | | |
| 1 | 74.31 | 249.01 | 452.90 | 0.30 |
| | 83.26 | 263.71 | 460.91 | 0.33 |
| 2 | 70.23 | 260.74 | 461.82 | 0.40 |
| | 74.66 | 279.32 | 519.19 | 0.32 |
| 3 | 66.60 | 251.32 | 453.66 | 0.44 |
| | 56.01 | 225.80 | 436.72 | 0.49 |
| 4 | 55.41 | 222.03 | 429.85 | 0.64 |
| | 55.01 | 215.57 | 433.20 | 0.69 |
| Minimum | 51.01 | 222.03 | 429.85 | 0.30 |
| Maximum | 83.26 | 279.32 | 519.19 | 0.69 |
| **Average** | **67.19** | **250.40** | **456.03** | **0.45** |
| Std. Dev | 10.61 | 19.03 | 28.39 | 0.15 |
| RSD% | 15.80 | 7.60 | 6.23 | |

**Table 3**

| Samples | Acid number (mg/g) | |
|---|---|---|
| | Process at 50°C | Process at 20°C |
| 1 | 0.05 | 0.33 |
| 2 | 0.05 | 0.29 |
| 3 | 0.06 | 0.35 |

## Claims

1. A process for preparing a sprayable oily liquid formulation comprising a mixture comprising, or alternatively consisting of:
- (a) one or more liposoluble vitamins of group D,
(b) a triglyceride of medium chain acids chosen among triglycerides of capric acid and/or caprylic acid, and
(c) a medium chain triglyceride oil (MCT oil) having a lower content of C8 and/or C10 fatty acids than that of (b),
said process comprising the steps of:
i. - dissolving the liposoluble vitamin of group D (a) in the medium chain triglyceride oil (c) heated to a temperature of 40°C to 60°C, under stirring until the disappearance of the bottom body so that to obtain an oily solution and vitamin;
ii. - adding the triglyceride of medium chain acids (b) to said oily solution and vitamin;
wherein said triglyceride of capric acid and/or caprylic acid (b) and said medium chain triglyceride oil (c) are present in a weight ratio of about 1:1;
iii. cooling the obtained solution to a room temperature of 20°C under stirring.

2. The process according to claim 1, wherein said triglyceride of medium chain acids is chosen among triglycerides having the following characteristics:
- caprylic acid (C8) 50% to 80%; preferably at least 59%, and capric acid (C10) 20% to 50%; preferably at least 40%;
- the sum of saturated fatty acids (C8+C10) is of 96% or above, preferably of 98% or above or above 99.5%;
- said triglycerides have a higher amount of caprylic acid (C8) than capric acid (C10) of a weight amount of 10% to 40%, preferably of 15% to 35%.

3. The process according to one of the claims 1-2, wherein said liposoluble vitamin of group D is chosen between vitamin D2 and/or vitamin D3.

4. The process according to one of the claims 1-3, wherein said formulation is an oily solution and has a density value of 0.92 g/cm³ to 0.98 g/cm³ at 25°C; preferably of 0.94 g/cm³ to 0.96 g/cm³ at 25°C.

5. The process according to claim 1, wherein said medium chain triglyceride oil is heated to a temperature of 45°C to 55°C, e.g. 50°C.

## Patentansprüche

1. Verfahren zur Herstellung einer zerstäubaren, öligen, flüssigen Formulierung, umfassend eine Mischung, umfassend oder alternativ bestehend aus:
-
(a) einem oder mehreren fettlöslichen Vitaminen der Gruppe D,
(b) einem Triglycerid von mittelkettigen Säuren, das unter den Triglyceriden von Caprinsäure und/oder Caprylsäure ausgewählt ist, und
(c) einem mittelkettigen Triglyceridöl (MCT-Öl) mit einem niedrigeren Gehalt an C8- und/oder C10-Fettsäuren als dem von (b),
wobei das Verfahren die folgenden Schritte umfasst:
i. - Lösen des fettlöslichen Vitamins der Gruppe D (a) in dem mittelkettigen Triglyceridöl (c), das auf eine Temperatur von 40°C bis 60°C erhitzt wurde, unter Rühren bis zum Verschwinden des Unterkörpers, um eine ölige Lösung und Vitamin zu erhalten;
ii. - Hinzufügen des Triglycerids von mittelkettigen Säuren (b) zu der öligen Lösung und Vitamin;
wobei das Triglycerid von Caprinsäure und/oder Caprylsäure (b) und das mittelkettige Triglyceridöl (c) in einem Gewichtsverhältnis von etwa 1:1 vorliegen;
iii. Abkühlen der erhaltenen Lösung auf eine Raumtemperatur von 20°C unter Rühren.

2. Verfahren nach Anspruch 1, wobei das Triglycerid von mittelkettigen Säuren aus Triglyceriden mit den folgenden Eigenschaften ausgewählt ist:
- Caprylsäure (C8) 50% bis 80%; vorzugsweise mindestens 59% und Caprinsäure (C10) 20% bis 50%; vorzugsweise mindestens 40%;
- die Summe der gesättigten Fettsäuren (C8+C10) 96% oder höher, vorzugsweise 98% oder höher oder höher als 99,5% beträgt;
- die Triglyceride eine höhere Menge an Caprylsäure (C8) als Caprinsäure (C10) in einer Gewichtsmenge von 10% bis 40%, vorzugsweise von 15% bis 35%, aufweisen.

3. Verfahren nach einem der Ansprüche 1 bis 2, wobei das fettlösliche Vitamin der Gruppe D aus Vitamin D2 und/oder Vitamin D3 ausgewählt ist.

4. Verfahren nach einem der Ansprüche 1 bis 3, wobei die Formulierung eine ölige Lösung ist und einen Dichtewert von 0,92 g/cm³ bis 0,98 g/cm³ bei 25°C, vorzugsweise von 0,94 g/cm³ bis 0,96 g/cm³ bei 25°C, aufweist.

5. Verfahren nach Anspruch 1, wobei das mittelkettige Triglyceridöl auf eine Temperatur von 45°C bis 55°C, z.B. 50°C, erhitzt wird.

## Revendications

1. Procédé de préparation d'une formulation liquide huileuse pulvérisable, comprenant un mélange comprenant, ou sinon constituée :
- (a) d'une ou plusieurs vitamines liposolubles du groupe D,
(b) d'un triglycéride d'acides à chaîne moyenne choisi parmi les triglycérides d'acide caprique et/ou d'acide caprylique, et
(c) d'une huile de triglycéride à chaîne moyenne (huile TCM) ayant une teneur en acides gras C8 et/ou C10 inférieure à celle de (b),
ledit processus comprenant les étapes de :
i. - dissoudre la vitamine liposoluble du groupe D (a) dans l'huile de triglycéride à chaîne moyenne (c) chauffée à une température de 40 °C à 60 °C, sous agitation jusqu'à la disparition du corps de fond de sorte à obtenir une solution huileuse et la vitamine ;
ii. - ajouter le triglycéride d'acides à chaîne moyenne (b) à ladite solution huileuse et à la vitamine ;
dans lequel ledit triglycéride d'acide caprique et/ou d'acide caprylique (b) et ladite huile de triglycéride à chaîne moyenne (c) sont présents dans un rapport pondéral d'environ 1:1 ;
iii. refroidir la solution obtenue à une température ambiante de 20 °C sous agitation.

2. Procédé selon la revendication 1, dans lequel ledit triglycéride d'acides à chaîne moyenne est choisi parmi les triglycérides ayant les caractéristiques suivantes :
- acide caprylique (C8) de 50 % à 80 % ; de préférence au moins 59 %, et acide caprique (C10) 20 % à 50 % ; de préférence au moins 40 % ;
- la somme des acides gras saturés (C8+C10) est de 96 % ou plus, de préférence de 98 % ou plus ou de plus de 99,5 % ;
- lesdits triglycérides ont une quantité plus élevée d'acide caprylique (C8) que d'acide caprique (C10) d'une quantité pondérale de 10 % à 40 %, de préférence de 15 % à 35 %.

3. Procédé selon l'une des revendications 1-2, dans lequel ladite vitamine liposoluble du groupe D est choisie entre la vitamine D2 et/ou la vitamine D3.

4. Procédé selon l'une des revendications 1-3, dans lequel ladite formulation est une solution huileuse et a une valeur de densité de 0,92 g/cm³ à 0,98 g/cm³ à 25 °C ; de préférence de 0,94 g/cm³ à 0,96 g/cm³ à 25 °C.

5. Procédé selon la revendication 1, dans lequel ladite huile de triglycéride à chaîne moyenne est chauffée à une température de 45 °C à 55 °C, par exemple à 50 °C.
